# EUROPEAN PATENT SPECIFICATION

(11) **EP 3 283 888 B1**
(45) Date of publication and mention of the grant of the patent: **10.06.2020**
(21) Application number: 16720906.3
(22) Date of filing: 14.04.2016
(51) Int. Cl.: B01L 3/00, G01N 27/00

(54) **A METHOD FOR IN VITRO DIAGNOSIS OF ALLERGY AND RELATED DEVICE**
VERFAHREN ZUR IN-VITRO-DIAGNOSE VON EINER ALLERGIE UND ZUGEHÖRIGE VORRICHTUNG
PROCÉDÉ DE DIAGNOSTIC IN VITRO DE L'ALLERGIE ET DISPOSITIF ASSOCIÉ

(30) Priority: 15.04.2015 IT FI20150110
(43) Date of publication of application: 21.02.2018
(73) Proprietor: Azienda Ospedaliero-Universitaria Meyer, 50139 Firenze (IT); Universita' Degli Studi di Firenze, 50121 Firenze (IT); Università degli Studi di Siena, 53100 Siena (IT)
(72) Inventor: PUCCI, Neri, 50135 Firenze (IT); ROSSI, Maria Elisabetta, 50131 Firenze (IT); MORI, Francesca, 53036 Poggibonsi (Siena) (IT); MUGNAINI, Marco, 50132 Firenze (IT); VIGNOLI, Valerio, 50137 Firenze (IT); FORT, Ada, 50141 Firenze (IT); NOVEMBRE, Elio, 50122 Firenze (IT)
(74) Representative: Brazzini, Silvia
(86) International application number: PCT/IB2016/052131
(87) International publication number: WO 2016/166699

(56) References cited:
- US-A1- 2010 222 224
- HUANG ET AL: "Impedance sensing of allergen-antibody interaction on glassy carbon electrode modified by gold electrodeposition", BIOELECTROCHEMISTRY, ELESEVIER, AMSTERDAM, NL, vol. 70, no. 2, 3 April 2007 (2007-04-03), pages 257-262, XP022015480, ISSN: 1567-5394, DOI: 10.1016/J.BIOELECHEM.2006.10.002
- LIU HONGYUN ET AL: "Electrochemical immunosensors for antibodies to peanut allergen ara h2 using gold nanoparticle-peptide films.", ANALYTICAL CHEMISTRY 1 JUL 2010, vol. 82, no. 13, 1 July 2010 (2010-07-01), pages 5865-5871, XP002751967, ISSN: 1520-6882
- DANKE XU ET AL: "Label-Free Electrochemical Detection for Aptamer-Based Array Electrodes", ANALYTICAL CHEMISTRY, vol. 77, no. 16, 1 August 2005 (2005-08-01), pages 5107-5113, XP055082964, ISSN: 0003-2700, DOI: 10.1021/ac050192m

## Description

### Field of the Invention

The present disclosure refers to a method for the detection and quantification of specific immunoglobulins E (IgE) for an allergen of interest in a patient's serum sample, and to a device for achieving this method.

### State of the Art

Immunoglobulins are heterodimeric proteins composed of two heavy chains and two light chains. They can be functionally separated into variable domains that bind antigens and constant domains that specify effector functions, such as activation of complement or binding to Fc receptors. Each variable domain can be split into three regions of variability in the sequence called Complementarity-Determining Regions (CDRs), and four regions having a relatively constant sequence called the framework regions.

The binding between the antigen and the antibody's variable domain is weak and essentially non-covalent. Electrostatic interactions, hydrogen bonds, van der Waals forces, and hydrophobic interactions are all known to be involved depending on the interaction sites (Absolom D.R. et al., 1986 CRC Critical Reviews in Immunology 6(1):1-46). There are five main classes of heavy chain constant domains. Each class defines the IgM, IgG, IgA, IgD, and IgE isotypes. On their turn the IgG isotype can be split into four subclasses, that are IgG1, IgG2, IgG3, and IgG4, each of them having its own biologic properties. Similarly, the IgA isotype can be split into IgA1 and IgA2 (Schroeder I. et al. J. Allergy Clin. Immunol. 2010 Feb; 125 (2 Suppl 2):S41-52).

The IgE is a very potent immunoglobulin, although it is present at the lowest serum concentration and has the shortest half-life. IgE is associated with hypersensitivity and allergic reactions, as well as with the response to parasitic worm infections. IgE binds with extremely high affinity to the receptor known as FcεRI, the high-affinity receptor for the Fc region of immunoglobulin E, which is expressed on mast cells, basophils, Langerhans cells, and eosinophils. Circulating IgE upregulates the FcεR expression on these cells. The combination of strong binding and upregulation of FcεR expression contributes to the remarkable potency of this immunoglobulin. IgE was initially identified in 1967 as the reagin that mediates type I hypersensitivity (see for example Ishizaka K. et al. J. Allergy 37 (1967), pp. 169-172).

In 1972 the first commercial assay for allergen-specific IgE called the RAST = Radio Allergic Sorbent Test, was developed (Wide L. et al. Lancet 2 (1967) pp. 1105-1109). The original RAST test was configured as a test carried out on a paper allergosorbent disc to which many different allergens of different specificities were covalently coupled. Serum was incubated with this solid-phase allergen, permitting antibodies of all isotypes to bind thereto. After a buffer wash to remove unbound proteins, bound IgE antibody was detected with radio-iodinated polyclonal antihuman IgE. After this second incubation and a second buffer wash, bound radioactivity was quantified in a gamma counter. The entire test required three days. The quantity of counts per minute bound was proportional to the amount of IgE antibody specifically bound to the immobilized allergen.

In more recent years, the improvements of technology in the solid-phase component has led to the development of the ImmunoCAP® system (Pharmacia Diagnostics) for *in vitro* testing. This test uses a three-dimensional cellulose solid phase as as a replacement of the two-dimensional paper disks of the modified RAST. As a result, the required incubation times are shorter. These newer IgE antibody assays use a larger number of allergen extracts, also having higher quality, for preparing the related allergosorbents. New matrix materials such as the cellulose sponge used in the ImmunoCAP® System have enhanced the binding capacity, while reducing the nonspecific binding properties of allergosorbents. Various polyclonal and monoclonal anti-lgE detection antibody combinations insure maximal assay sensitivity and specificity for human IgE. Nonisotopic labels have increased the shelf life of reagents and have made the assays more user-friendly and safer to perform. Moreover, automation has improved assay precision and reproducibility to the level where some IgE antibody assays on auto-analysers require only single measurements to insure accurate results. Calibration systems used in the second-generation IgE antibody assays have employed a common strategy in which a heterologous total serum IgE curve is used to convert allergen-specific IgE assay response data into quantitative dose estimates of IgE antibody. The entire test takes about six hours for completion.

Two further diagnostic assays for the determination in laboratory of specific serum IgE have been developed. One is called Immulite® system (Siemens, Germany) and uses a biotinylated allergen that is bound to an avidin solid phase. The other one is the HyTech-288 system (Hycor/Agilent Technologies, California, US) that uses a cellulose wafer on which the allergen is covalently coupled.

The above mentioned three systems are all performed on auto-analysers to maximize precision and minimize the turnaround time. They all use nonisotypically labelled antihuman IgE and are calibrated by means of interpolation of response data from a heterologous total serum IgE calibration curve that has been referenced to the World Health Organization (WHO) total IgE serum standard (see "Analytical performance characteristics and clinical utility of immunological assays for human immunoglobulin E antibodies of defined allergen specificities". Clinical and Laboratory Standards Institute (CLSI), Wayne, Pa (2009) Approved guideline I/LA20).

These serologic assays have evolved from dichotomous qualitative measures of IgE antibody (e.g. positive vs. negative) to a more quantitative status. In fact, clinical research has shown that, in certain cases, the level of allergen-specific IgE antibody might be predictive of a positive allergen challenge test, such as relates to food allergy, and of the presence of more severe disease (e.g. wheeze) in children with asthma (see Eckman J. et al. Allergy Asthma Clin. Immunol., 5 (2009), pp. 2-8; and Simpson A. et al. J. Allergy Clin. Immunol., 116 (2005), pp. 744-749). However, serum IgE concentrations do not accurately predict the severity of allergic reaction, but do reflect the likelihood of an allergic reaction of variable intensity.

### Summary of the Invention

Subject of the present disclosure is therefore to a method for *in vitro* diagnosis of allergic disease and a device for achieving this method, that solve the technical problems highlighted above for the known tests, allowing optimizing the times required for carrying out the test, and also lowering the costs in terms of reagents and instruments needed.

A further subject of the present disclosure is to provide a method and related device for *in vitro* diagnosis of allergic disease, which allows performing in a very accurate way quantitative measurements of the immunoglobulins E (IgE) specific for the allergens of interest.

These and further subjects are achieved by the method for *in vitro* diagnosis of allergic disease and related device according to the present invention, whose essential features are defined in the independent claims here attached. Further important characteristics of the method and device according to the invention are defined in the dependent claims.

### Brief description of the figures

The characteristics and advantages of the method and device for diagnosis of allergies according to the present invention, will be clearly illustrated in the following exemplary and not limiting description of their embodiments, also with reference to the attached figures wherein:
- Figures 1 and 2 respectively show a perspective and a cross-sectional view of a preferred embodiment of the device according to the invention;
- Figures 3a), 3b), 3c), 4, 5a), 5b), 5c), 6a), 6b) and 6c) show the impedance curves obtained by using the device of the invention by measuring the impedance values of various patients' sera as described in the following Examples 1 to 4.

### Detailed Description of the Invention

As said above, the immunoglobulin E (IgE) is an immunoglobulin present in human serum and associated with hypersensitivity and allergic reactions, so that the allergy tests are generally based on the dosage of IgE in a patient's blood sample in response to a certain allergen. The aim of the present invention is to provide an alternative and more efficient method for diagnosis of allergic diseases by measuring the impedance values of a patient's serum sample before and after its contact with an allergen of interest.

The interaction between the immunoglobulin E and the allergen occurs through a bonding mechanism, as for any other antibody/antigen interactions. Following this interaction some of the free protons/electrons of the immunoglobulin structure come closer to the allergen structure, thus enhancing or respectively reducing the impedance value of the serum under testing. In other words, the higher is the difference in the impedance values measured, the higher is the interaction between the IgE and the allergen under testing.

Now the Applicants have developed a novel device based on the measurement of the impedance values in serum samples that allows evaluating the allergic reactivity of a patient in response to an allergen by testing a patient's serum sample in a very easy and fast way, by measurements that prove to be reproducible and repeatable over time.

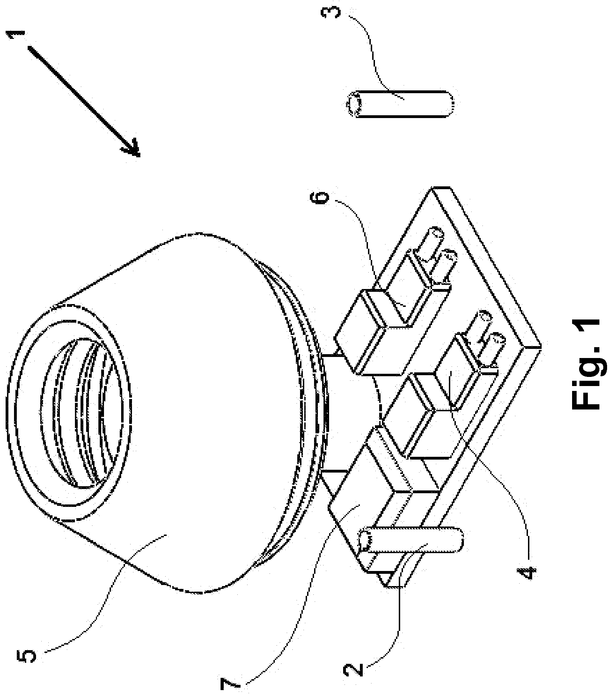

With particular reference to the figure 1, the device 1 for the diagnosis of allergic disease according to the invention comprises:
- a container 2 for an allergen of interest for which specific IgE are to be detected in a patient's serum sample;
- a measurement unit 3 comprising an appropriate support for allergen/serum samples and electrodes for the generation of AC electric signals on the support;
- a pump 4 for taking a sample of allergen from the container 2 and pumping it towards the measurement unit 3 through suitable pipes;
- a container 5 for a patient's serum to be tested, optionally comprising a centrifuge and filtering device for the treatment of the patient's whole blood and its separation for obtaining a serum sample to be tested;
- a pump 6 for taking a patient's serum sample to be tested from the container 5 and pump it towards the measurement unit 3 through suitable pipes;
- a processing unit 7 associated to the measurement unit 3 is able to collect and process the data coming from said unit 3 to obtain corresponding impedance values of the samples tested, and send them to a display or to a remote station for a further processing and display of data.

By remote station is meant here in particular a personal computer or a smart device that, according to a preferred embodiment of the invention, may also generate set up messages for the measurement unit 3 and/or the processing unit 7 at different frequencies and amplitudes.

The above said container 2 may be a container able to separately hold different allergens, from which the allergen of interest is selected from time to time.

The measurement unit 3 comprises a support that is designed to have the minimum resistance and it consists preferably of a pad covered by a noble metal, such as gold or platinum, forming a couple of electrodes, in order to minimize any oxidation process and interaction of the pad with the biological materials under testing. After every drop addition or test, the pad is preferably cleaned with an alcoholic solution thus reducing the risks of interference between one measurement and the subsequent measurements. The support according to the present invention preferably consisting of a pad covered by a noble metal to form the couple of electrodes, is therefore nonspecific towards the allergen under analysis, i.e. it does not requires any modifications or functionalization of the measurement electrodes depending on the allergen to be investigated. Therefore, with a same device according to the present invention, different allergens may be tested without the need to make any modifications to the measurement unit and to the support when moving from an allergen to another. In other words, the present device may be used for the diagnosis of different allergic diseases, associated to different allergens, without the need to change the measurement unit, and in particular the measurement electrodes, that are not allergen-specific.

The impedance is generally defined as the total opposition that a circuit, or more in general an object, offers to the flow of alternating current (AC) at a given frequency. Many methods are known for the measurement of impedance; the most appropriate can be selected according to the measurements requirements and conditions, such as frequency coverage or measurements accuracy required. The measurement unit 3 of the present device, and the associated processing unit 7 can be built by a proper hardware structure with a software and an interdigitated baseplate or a pad stripe made of a noble metal, such as gold, as a support for the samples to be tested. Suitable examples of an impedance measurement unit 3 and associated processing unit 7 are for instance those systems disclosed in "AGILENT IMPEDANCE MEASUREMENT HANDBOOK" 4th edition; or disclosed by Ackman J.J. et al. 1984 Critical Reviews in Biomedical Engineering 11 (4), pp. 281-311; Chen S.W. tt al. 2012 Biosensors and Bioelectronics 33(1), pp. 196-203; Dvorskij, V.Ya. et al. 1998 Izvestiya VUZ: Radioelektronika 41 (7), pp. 75-77.

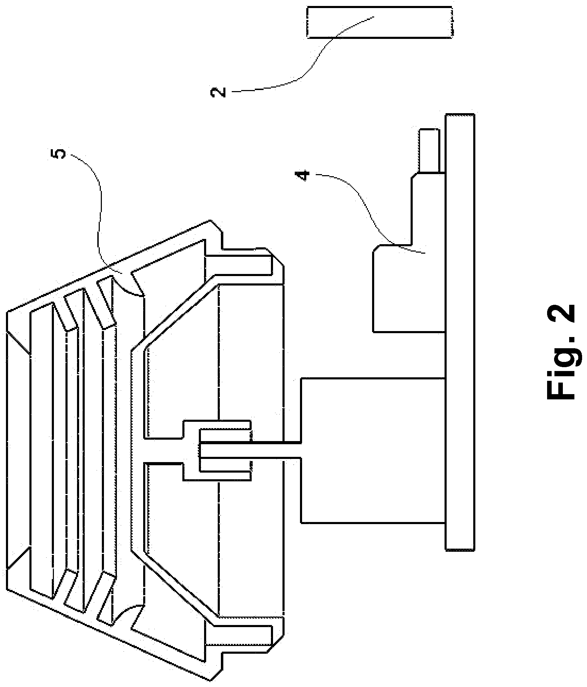

According to a preferred embodiment of the present invention, the container 5 comprises a centrifuge and a filtering device downstream of it, so that a patient's blood sample as taken from the patient can be used directly for the testing in the present device; this whole blood being processed by the centrifuge and by the filter to obtain a serum containing the IgE of interest, that are then tested for impedance with an allergen according to the present method. With particular reference to the Figure 2, an example of the container 5 and of its inner structure is illustrated; containers of different forms and dimensions are however possible.

In the present invention the impedance measurements are carried out in alternate current (AC) regime at a frequency ranging from 10 Hz up to 100 kHz with a resolution of at least 1 Hz, so as to minimize the interaction and the biasing effects of those substances still present in serum, that could become polarized if biased with a constant voltage; in this way the properties of the serum under test are preserved at highest grade.

The flow of serum and allergens in the device of the invention are assisted by pumps that let then flow through pipelines and regulate their deposition on the support in the measurement unit 3. The amount of serum to be tested and of allergen in the present device is regulated to be from 100 uL to 40 mL. The impedance values measured for such volumes can range from 500 kΩ to 5 Ω.

A dedicated software in the processing unit 7 is able to set the proper measurement parameters to perform the impedance measurements in a range of frequency to perform a frequency sweep or at a single frequency according to the test needs, and the amplitude of the signals on the sample may be set for instance between 200mVₚₚ and 2 Vₚₚ. The data collected from the measurements can then be collected through a shared or dedicated bus; preferably, a I2C communication protocol is used to set up the impedance measurement unit and the collection of data obtained.
The present method for *in vitro* diagnosis of allergic disease comprises the following steps:
- contacting an allergen containing solution with a support provided with electrodes;
- measuring impedance of said allergen containing solution, obtaining a first impedance value;
- adding a patient's serum to said allergen containing solution onto said support provided with electrodes;
- measuring impedance of said serum added to said allergen containing solution, obtaining a second impedance value;
- evaluating the difference between said second and said first impedance values, this difference being correlated to the concentration of IgE specific to said allergen in said serum.
The above said first impedance value obtained for the allergen containing solution represents a baseline of the present measurement method, corresponding to the base impedance at the frequency and amplitudes selected for the measurement.
The variation of impedance when serum is added is correlated with the concentration of IgE present in serum and specific to the allergen, and can gives a measure of the interaction between the allergen and the specific IgE. In fact, by addition of further amounts of serum and measuring impedance, a curve may be obtained whose slope gives a measure of the reaction rate and of the dynamics of the allergen-protein interaction. In other words, the present method may comprise repeated cycles of the above indicated steps, wherein the measurement of impedance of the serum added to the allergen solution is repeated at determined time intervals, so obtaining several second impedance values at different times, whose difference with the first impedance value of the allergen-containing solution is correlated with the variation in time of the concentration of IgE present in the serum under investigation and specific for the allergen at issue.
The experiments carried out by the Applicants and described in the following have shown that the present method is allergen-dependent, i.e. the impedance measurements depend on the concentration of allergen used. Optimal measurements conditions include a concentration of the allergen ranging between 10 mg/mL and 150 mg/mL.

Advantages of the present device are: a great accuracy, always within 1% of the full scale measurement; a great ease of operations and use of the device; the possibility to study the kinetics of the reaction IgE/allergen that is not possible by any other assay known to the Applicants.

This kind of information on the reaction kinetics between specific IgE and allergen is in fact an important parameter for the evaluation in a subject of the severity of a certain allergic form of the subject themselves, and in particular of the possibility for the subject to suffer anaphylaxis and, in the most serious cases, the so called anaphylactic shock, due to a certain allergen. As a matter of fact the anaphylaxis is a rapid-onset, severe allergic reaction; within the present diagnostic method, therefore, a dynamic measurement of impedance in a subject's serum, as described above, that highlights a strong initial variation of the detected impedance, can indicate a predisposition of the subject to anaphylactic episodes. Vice versa, a very slow variation of the impedance, even if tending to reach higher absolute values for relatively long times, can indicate a lack of predisposition in the subject to anaphylactic episodes. See for instance Simons F.E.R. et al. World Allergy Organization Guidelines for the Assessment and Management of Anaphylaxis, J. Allergy Clin. Immunol. 2011: 127, 587-93.

A further important advantage of the present device and method is the much lower cost for reagents and instruments with respect to the known techniques: in the present method the only treatment made before the impedance measurement is the preparation of serum from the patient's sample of whole blood, which requires just a centrifuge and optionally a filter, while no markers nor immobilization/functionalization agents nor anti-human IgE are needed in the present method.

In particular, compared to the RAST/ImmunoCap(R) techniques, the present method is much simpler because it minimizes the possible loss of allergenic epitopes in the preparation of the solid phase, it is much faster because the entire test with the present method just takes 5 minutes to arrive to completion, and it is cheaper than the immunoenzymatic tests, the only costs being for the obtainment of serum and for the allergen.

Finally, a further advantage of the present method is that it allows studying the kinetics of the reaction antigen(allergen)/antibody(IgE) by measuring the slope interaction dynamics. The derivative measurement indicates, as in the measurements of gas sensors, the kinetics of the reaction. The so measured value is therefore an indication of the time constant of the reaction, supposed of the first order.

### EXPERIMENTAL PART

### Measurement of the allergic reactivity in serum from subjects allergic to casein of cow's milk by using the device of the invention

### Example 1

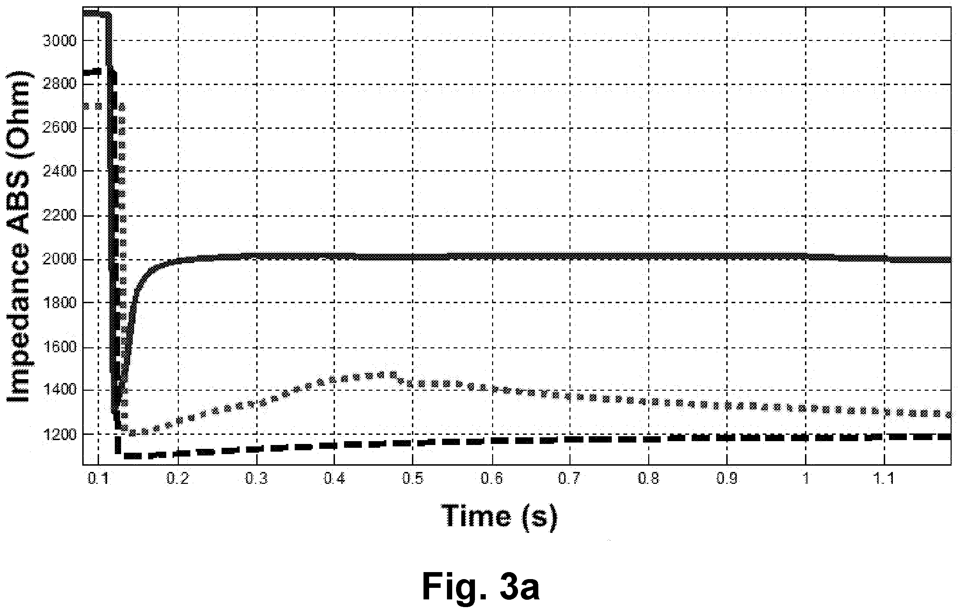

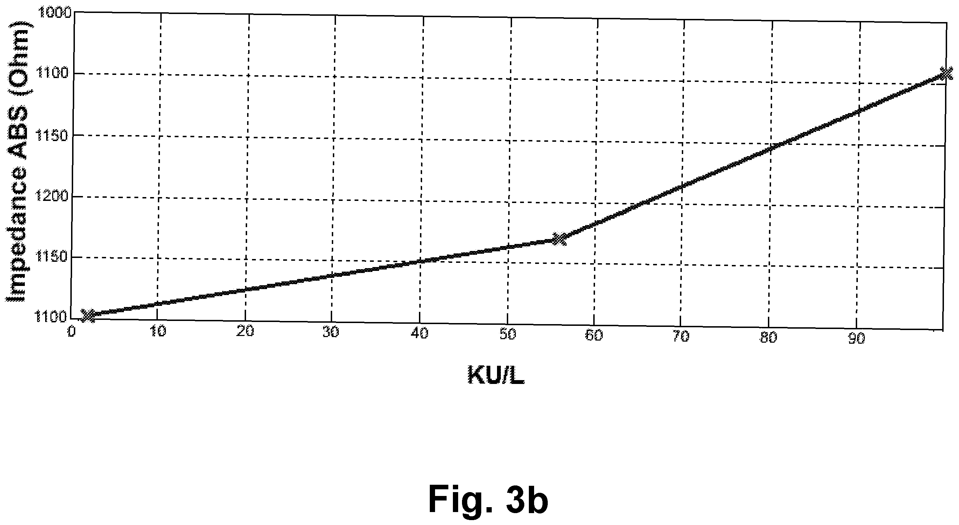

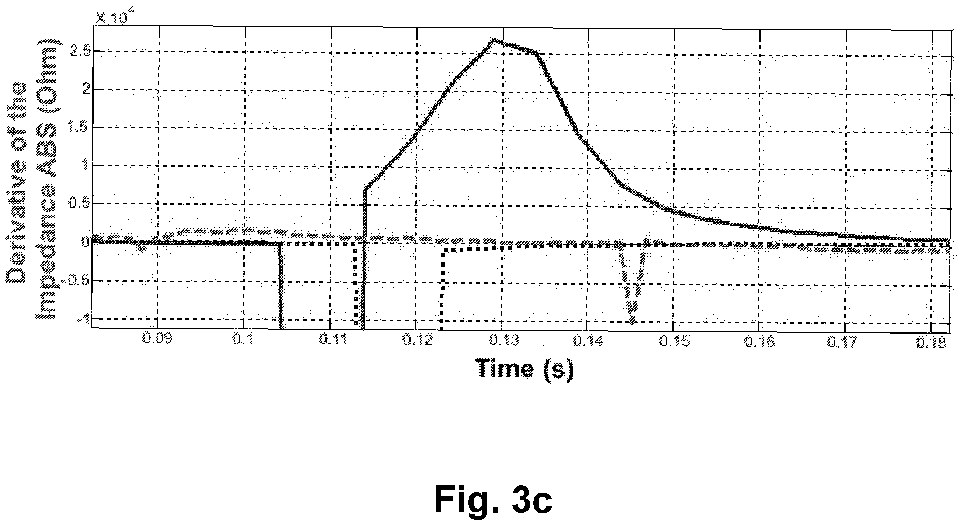

Initially sera were examined that were taken from three patients having different levels of IgE specific for casein, detected with the known ImmunoCap® method. The measurement of the impedance values of these sera was carried out with the device of the present invention, and results are depicted in Figure 3a, wherein the curve (--) corresponds to the patient with a higher value of specific IgE for casein (> 100 KU /I), the curve (- - -) was obtained for the patient having intermediate values for the IgE specific for casein (58 KU/I), and finally the curve (---) was obtained for the patient having a value of IgE specific for casein of 2 KU/L. The trend of the three impedance curves in Figure 3 is correlated to the values of serum specific IgE.

These measurements and the following are all carried out on drops of the samples to be tested, each drop having a volume of 15 mL, whereas the specific frequency used was of 30 kHz.

In Figure 3b are indicated the steady state values of the responses of same three patients mentioned above having IgE values of >100, 58 and 2 respectively.

In Figure 3c the derivative of the reaction dynamics is represented by using the same curves representation for the three patients as described above. For the allergic patients a correlation seems to exist between positive derivatives after the allergen addition and the grade of the response to the allergen.

### Example 2

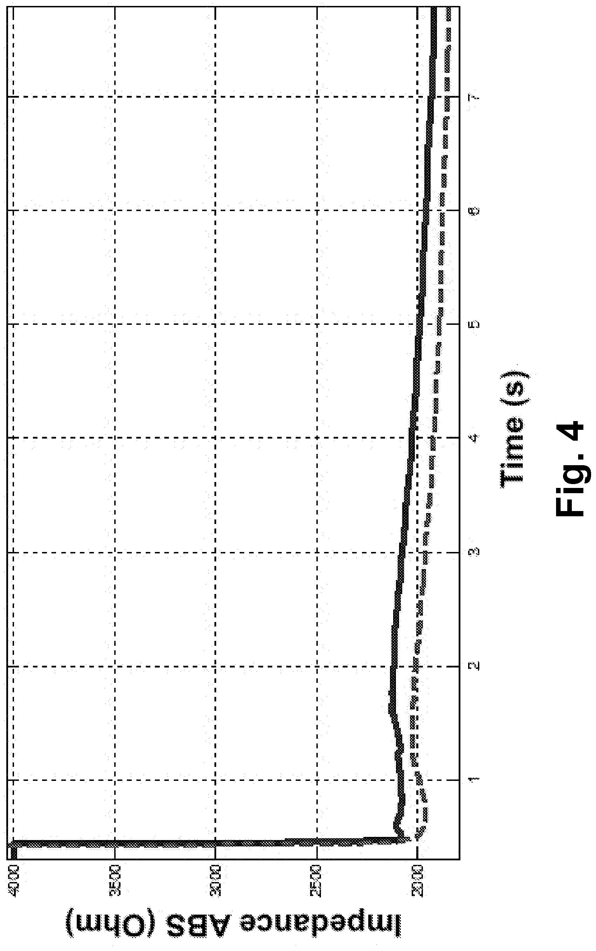

For the same three patients the repeatability of the impedance measurement with the present device was then investigated, by carrying out for each of the three patients two identical trials using the patient's serum and the allergen, i.e. casein. Results of these trials are illustrated in Figure 4 wherein the trial with casein is represented by a curve (-(--), and the trial with the patient's serum is represented by a curve (--). It is worth noting that the results obtained in the trials carried out for each patient are substantially overlapping.

### Example 3

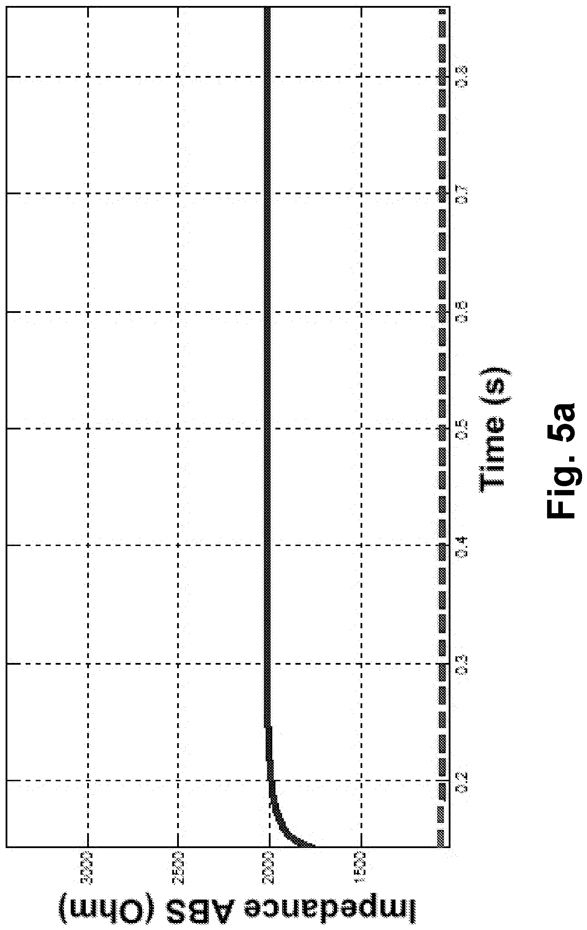

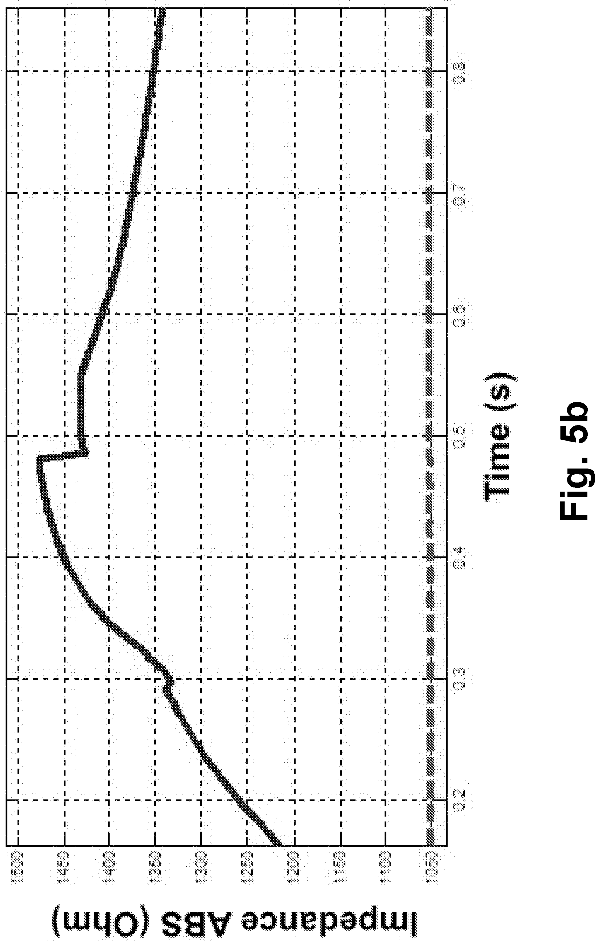

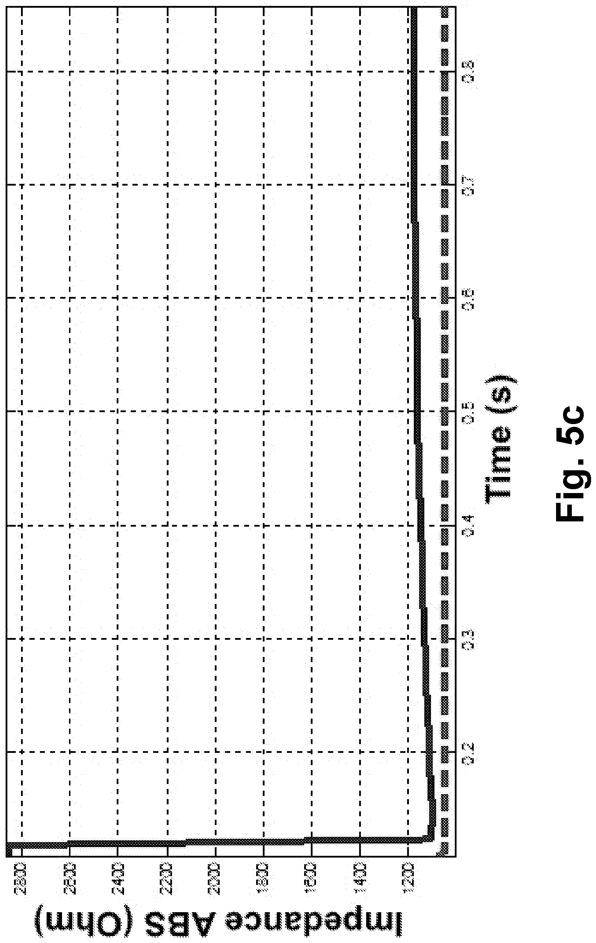

For the same three patients mentioned above the impedance measurements with the device of the invention were repeated by recording the impedance curves serum-allergen and serum-serum for each of the three patients; their comparison gives a measure of the difference of impedance between allergen (- - -) and serum (--). The results are illustrated in Figures 5 from a) to c).

### Example 4

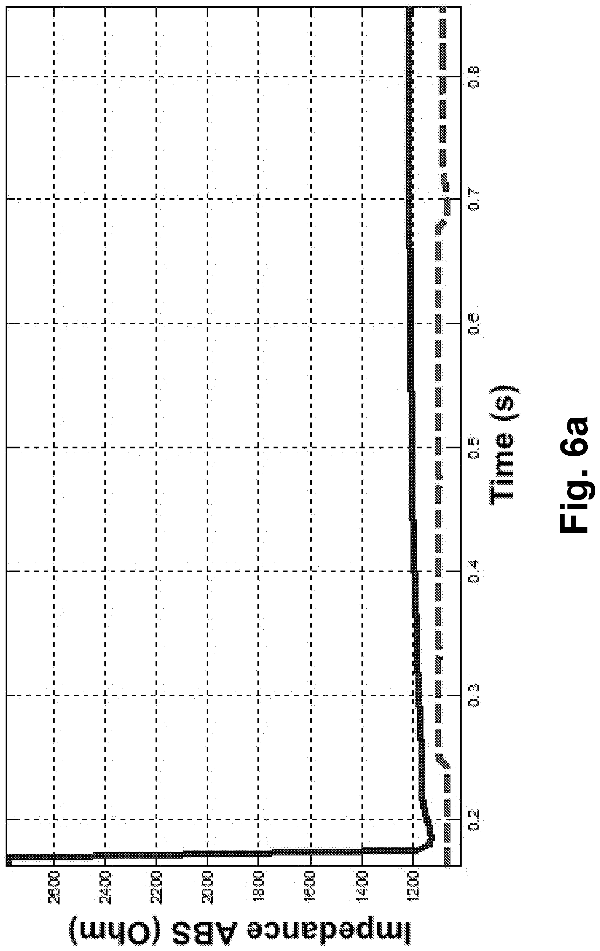

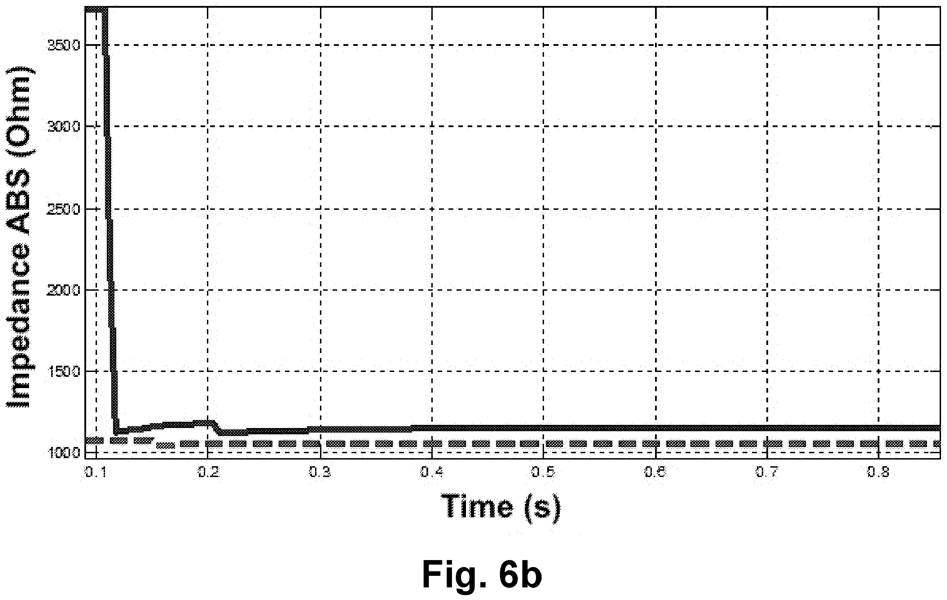

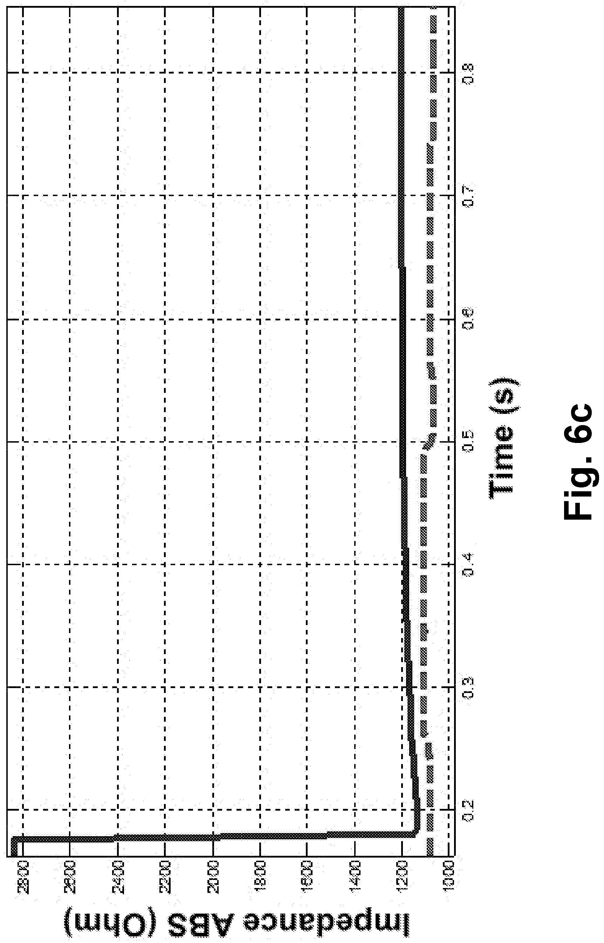

Finally, sera from three patients without IgE specific for casein and suffering from vernal keratoconjunctivitis, thus being patients for which casein allergy is not possible, were tested as a negative control, to verify that the impedance measurements with the present device do not give any response in these cases. In these experiments the patient's sera were put in contact with the casein and with their sera, obtaining in both cases a lack of response. Results of the two experiments are illustrated in Figures 6 from a) to c).

In conclusion, from the experiments carried out and described above it is evident that:
- there is a correlation between the values of IgE specific for casein and the trend of the impedance curves;
- the diagnostic test based on measurements with the present device is reproducible in allergic patients;
- for healthy subjects, not allergic to casein, no response is detected in the impedance measurement of the present test.

## Claims

1. A method for *in vitro* diagnosis of an allergic disease comprising the following steps:
- contacting an allergen containing solution with a support provided with electrodes, consisting of a pad coated by a noble metal forming a couple of said electrodes;
- measuring impedance of said allergen containing solution, obtaining a first impedance value;
- adding a patient's serum to said allergen containing solution onto said support provided with electrodes;
- measuring impedance of said serum added to said allergen containing solution, obtaining a second impedance value;
- evaluating the allergic reactivity of said patient in response to said allergen by evaluating the difference between said second and said first impedance values, this difference being correlated to the concentration of IgE specific to said allergen in said serum and to the grade of the response to allergen, while no response to said allergen is detected for healthy subjects.

2. The method according to claim 1, wherein said couple of electrodes are made of gold or platinum.

3. The method according to any one of the preceding claims, wherein said support provided with electrodes is washed after every addition to reduce the risks of interference between subsequent measurements.

4. The method according to any one of the preceding claims, wherein said measuring of impedance are carried out in alternate current (AC) regime at a frequency ranging from 10 Hz to 100 kHz with a resolution of at least 1 Hz.

5. The method according to any one of the preceding claims, wherein said allergen containing solution has a concentration of allergen ranging from 10 mg/mL to 150 mg/mL.

6. The method according to any one of the preceding claims, wherein said step of measuring the impedance of the serum added to the allergen containing solution and said step of evaluating the difference between said first and said second impedance value are repeated at time intervals to evaluate the variation in time of said difference, which is correlated with the variation in time of the concentration of said specific IgE.

7. A device (1) for the *in vitro* diagnosis of an allergic disease according to a method as defined in claims 1-6, said device comprising:
- a container (2) for an allergen of interest for which specific IgE are to be detected in a patient's serum sample;
- a measurement unit (3) comprising an appropriate support for allergen/serum samples and electrodes for the generation of AC electric signals on the support, wherein said support is a pad coated by a noble metal forming a couple of electrodes;
- a pump (4) for taking a sample of allergen from the container (2) and pumping it towards the measurement unit (3) through suitable pipes;
- a container (5) for a patient's serum to be tested;
- a pump (6) for taking a patient's serum sample to be tested from the container (5) and pump it towards the measurement unit (3) through suitable pipes;
- a processing unit (7) associated to said measurement unit (3) and able to collect and process the data coming from said unit (3) to obtain corresponding impedance values of the samples tested, and send them to a display or to a remote station for a further processing and display of data.

8. The device according to claim 7, further comprising a centrifuge and filtering device in said container (5) for the treatment of the patient's whole blood and its separation for obtaining said serum sample to be tested.

9. The device according to any one of claims 7 or 8, wherein said container (2) is a container able to separately hold different allergens, from which an allergen of interest is selected from time to time.

10. The device according to claim 7, wherein said couple of electrodes are made of gold or platinum.

11. The device according to any one of claims 7-10, wherein the processing unit (7) comprises a dedicated software able to set the proper measurement parameters to perform the impedance measurements in a range of frequency to perform a frequency sweep or at a single frequency according to a test needs, and to set the amplitude of the signals on the sample.

## Patentansprüche

1. Verfahren zur in vitro Diagnose einer allergischen Erkrankung, das die folgenden Schritte enthält:
- in Kontakt Bringen einer ein Allergen enthaltenden Lösung mit einem mit Elektroden versehenen Träger, der aus einem Kissen besteht, das mit einem Edelmetall beschichtet ist, das ein Paar der besagten Elektroden bildet;
- Messen der Impedanz der das Allergen enthaltenden Lösung, wobei ein erster Impedanzwert erhalten wird;
- Hinzufügen des Serums eines Patienten zu der das Allergen enthaltenden Lösung auf dem Träger, der mit den Elektroden versehen ist;
- Messen der Impedanz des Serums, das zu der das Allergen enthaltenden Lösung hinzugefügt wurde, wobei ein zweiter Impedanzwert erhalten wird;
- Evaluierung der allergischen Reaktivität des Patienten in Abhängigkeit von dem Allergen durch Evaluieren des Unterschiedes zwischen den ersten und den zweiten Impedanzwerten, wobei dieser Unterschied zu der Konzentration von IgE korreliert wird, das für das Allergen in dem Serum und dem Grad des Ansprechens auf das Allergen spezifisch ist, während kein Ansprechen auf das Allergen für gesunde Subjekte detektiert wird.

2. Verfahren nach Anspruch 1, wobei das besagte Paar der Elektroden aus Gold oder Platin hergestellt ist.

3. Verfahren nach einem der vorhergehenden Ansprüche, wobei der Träger, der mit den Elektroden versehen ist, nach jeder Hinzufügung gewaschen wird, um die Risiken der Beeinträchtigung zwischen aufeinanderfolgenden Messungen zu reduzieren.

4. Verfahren nach einem der vorhergehenden Ansprüche, wobei die Messungen der Impedanz unter dem Regime eines Wechselstroms (AC) bei einer Frequenz, die im Bereich von 10 Hz bis 100 kHz liegt, mit einer Auflösung von wenigstens 1 Hz ausgeführt wird.

5. Verfahren nach einem der vorhergehenden Ansprüche, wobei die das Allergen enthaltende Lösung eine Konzentration des Allergens hat, die im Bereich von 10 mg/mL bis 150 mg/mL liegt.

6. Verfahren nach einem der vorhergehenden Ansprüche, wobei der Schritt des Messens der Impedanz des Serums, das zu der das Allergen enthaltenden Lösung hinzugefügt wurde, und der Schritt des Evaluierens des Unterschiedes zwischen dem ersten und dem zweiten Impedanzwert in Zeitintervallen wiederholt werden, um über die Zeit die Variation des Unterschiedes zu evaluieren, die mit der Variation der Konzentration des IgE's über die Zeit korreliert ist.

7. Vorrichtung (1) für die in vitro Diagnose einer allergischen Erkrankung gemäß einem Verfahren, wie es in den Ansprüchen 1 bis 6 definiert ist, wobei die Vorrichtung enthält:
- einen Behälter (2) für ein Allergen, das von Interesse ist, für welches das spezifische IgE in einer Probe des Serums eines Patienten detektiert werden soll;
- eine Messeinheit (3), die einen geeigneten Träger für Allergen/Serum-Proben und Elektroden für die Erzeugung von elektrischen AC-Signalen auf dem Träger enthält, wobei der Träger ein Kissen ist, das mit einem Edelmetall beschichtet ist, das ein Paar von Elektroden bildet;
- eine Pumpe (4) zum Nehmen einer Probe des Allergens aus dem Behälter (2) und dessen Pumpen hin zu der Messeinheit (3) durch geeignete Röhren;
- einen Behälter (5) für das Serum eines Patienten, das getestet werden soll;
- eine Pumpe (6) zum Nehmen einer Probe des Serums eines Patienten, das getestet werden soll, aus dem Behälter (5) und dessen Pumpen hin zu der Messeinheit (3) durch geeignete Röhren;
- eine Verabeitungseinheit (7), die mit der Messeinheit (3) verbunden ist und geeignet ist, die Daten, die von der Einheit (3) kommen, zu sammeln und zu verarbeiten, um entsprechende Impedanzwerte der getesteten Proben zu erhalten, und sie zu einer Anzeige oder einer entfernten Station für eine Weiterverarbeitung und Anzeige von Daten zu schicken.

8. Vorrichtung nach Anspruch 7, wobei ferner eine Zentrifuge und Filtervorrichtung in dem Behälter (5) für die Behandlung des gesamten Blutes des Patienten und seine Trennung enthalten sind, um die Serumprobe zu erhalten, die getestet werden soll.

9. Vorrichtung nach einem der Ansprüche 7 oder 8, wobei der Behälter (2) ein Behälter ist, der geeignet ist, verschiedene Allergene getrennt zu halten, von denen ein Allergen, das von Interesse ist, von Zeit zu Zeit ausgewählt wird.

10. Vorrichtung nach Anspruch 7, wobei das besagte Paar der Elektroden aus Gold oder Platin hergestellt ist.

11. Vorrichtung nach einem der Ansprüche 7 bis 10, wobei die Verarbeitungseinheit (7) eine dedizierte Software enthält, die geeignet ist, die richtigen Messparameter einzustellen, um die Impedanzmessungen in einem Frequenzbereich, um einen Frequenzdurchlauf auszuführen, oder bei einer einzelnen Frequenz gemäß Testerfordernissen auszuführen und die Amplitude der Signale auf der Probe einzustellen.

## Revendications

1. Un procédé de diagnostic in vitro d'une maladie allergique comprenant les étapes suivantes :
- mise en contact d'une solution contenant un allergène avec un support muni d'électrodes, consistant en une plaquette recouverte d'un métal noble formant un couple de dites électrodes ;
- mesure de l'impédance de ladite solution contenant l'allergène, pour obtenir une première valeur d'impédance ;
- ajout du sérum d'un patient à ladite solution contenant l'allergène sur ledit support muni d'électrodes ;
- mesure de l'impédance du dit sérum ajouté à ladite solution contenant l'allergène, pour obtenir une seconde valeur d'impédance ;
- évaluation de la réactivité allergique du dit patient au dit allergène par évaluation de la différence entre lesdites seconde et première valeurs d'impédance, cette différence étant corrélée à la concentration en l'IgE spécifique du dit allergène dans ledit sérum et au degré de la réponse à l'allergène, tandis qu'aucune réponse au dit allergène n'est détectée pour des sujets en bonne santé.

2. Le procédé selon la revendication 1, dans lequel ledit couple d'électrodes est en or ou en platine.

3. Le procédé selon l'une quelconque des revendications précédentes, dans lequel ledit support muni d'électrodes est lavé après chaque ajout pour réduire les risques d'interférence entre les mesures suivantes.

4. Le procédé selon l'une quelconque des revendications précédentes, dans lequel lesdites mesures d'impédance sont effectuées en régime de courant alternatif (CA) à une fréquence allant de 10 Hz à 100 Hz avec une résolution d'au moins 1 Hz.

5. Le procédé selon l'une quelconque des revendications précédentes, dans lequel ladite solution contenant un allergène a une concentration en allergène allant de 10 mg/ml à 150 mg/ml.

6. Le procédé selon l'une quelconque des revendications précédentes, dans lequel ladite étape de mesure de l'impédance du sérum ajouté à la solution contenant l'allergène et ladite étape d'évaluation de la différence entre ladite première et ladite seconde valeur d'impédance sont répétées à des intervalles de temps pour évaluer la variation dans le temps de ladite différence, qui est corrélée à la variation dans le temps de la concentration de ladite IgE spécifique.

7. Un dispositif (1) pour le diagnostic in vitro d'une maladie allergique selon un procédé tel que défini dans les revendications 1 à 6, ledit dispositif comprenant :
- un récipient (2) pour un allergène d'intérêt pour lequel des IgE spécifiques doivent être détectées dans un échantillon de sérum d'un patient ;
- une unité de mesure (3) comprenant un support approprié pour des échantillons d'un allergène/sérum et des électrodes pour générer des signaux électriques CA sur le support, ledit support étant une plaquette recouverte d'un métal noble formant un couple d'électrodes ;
- une pompe (4) pour prélever un échantillon d'allergène dans le récipient (2) et l'envoyer vers l'unité de mesure (3) via des conduits appropriés ;
- un récipient (5) pour le sérum d'un patient à tester ;
- une pompe (6) pour prélever un échantillon de sérum de patient à tester dans le récipient (5) et l'envoyer vers l'unité de mesure (3) via des conduits appropriés ;
- une unité de traitement (7) associée à ladite unité de mesure (3) et apte à collecter et traiter les données provenant de ladite unité (3) pour obtenir les valeurs d'impédance correspondant aux échantillons testés, et les envoyer à un afficheur ou une station à distance pour un traitement ultérieur et l'affichage de données.

8. Le dispositif selon la revendication 7, comprenant en outre un dispositif de centrifugation et de filtration dans ledit récipient (5) pour le traitement du sang total du patient et sa séparation pour obtenir ledit échantillon de sérum à tester.

9. Le dispositif selon l'une quelconque des revendications 7 et 8, dans lequel ledit récipient (2) est un récipient capable de contenir séparément différents allergènes, parmi lesquels un allergène d'intérêt est sélectionné de temps en temps.

10. Le dispositif selon la revendication 7, dans lequel ledit couple d'électrodes est en or ou en platine.

11. Le dispositif selon l'une quelconque des revendications 7 à 10, dans lequel l'unité de traitement (7) comprend un logiciel dédié capable de régler les paramètres de mesure appropriés pour effectuer les mesures d'impédance dans une plage de fréquences pour effectuer un balayage de fréquence ou à une seule fréquence selon un besoin de test, et pour régler l'amplitude des signaux sur l'échantillon.
